# EUROPÄISCHE PATENTANMELDUNG

(11) **EP 1 847 594 A2**
(43) Veröffentlichungstag der Anmeldung: **24.10.2007**
(21) Anmeldenummer: 07007929.8
(22) Anmeldetag: 19.04.2007
(51) Int. Cl.: C12N 5/06

(54) **Künstliches Lungensystem und dessen Verwendung**

(30) Priorität: 21.04.2006 DE 102006020494
(71) Anmelder: NovaLung GmbH, 72379 Hechingen (DE)
(72) Erfinder: Fischer, Stefan, 51503 Roesrath (DE); Matheis, Georg, 72393 Burladingen (DE)
(74) Vertreter: Laufer, Gabriele

(57) **Zusammenfassung**

Bei einem künstlichen Lungensystem (10) trennt eine Gasaustauschmembran (11) eine Blutseite (12) von einer Luftseite (14), wobei die Gasaustauschmembran auf der Blutseite (12) und der Luftseite (14) jeweils eine Fremdoberfläche (15, 16) aufweist, die auf der Blutseite (12) und/oder auf der Luftseite (14) mit biologischen Zellen (22, 21) besiedelt ist. Das künstliche Lungensystem kann zur Herstellung eines extrakorporalen oder implantierbaren Lungenunterstützungssystems oder zur Herstellung eines Lungenmodellsystems für die Untersuchung von Atemwegsbelastungen verwendet werden (Fig.).

## Beschreibung

Die vorliegende Erfindung betrifft ein künstliches Lungensystem mit einer Gasaustauschmembran, die eine Blutseite von einer Luftseite trennt, wobei die Gasaustauschmembran auf der Blutseite und der Luftseite jeweils eine Fremdoberfläche aufweist.

Die Erfindung betrifft ferner die Verwendung des künstlichen Lungensystems zur Herstellung eines extrakorporalen oder implantierbaren Lungenunterstützungssystems sowie zur Herstellung eines Lungenmodellsystems als Ersatz für Tiermodellsysteme bei toxikologischen Untersuchungen.

Ein künstliches Lungensystem der eingangs genannten Art, das als extrakorporales Lungenunterstützungssystem verwendet wird, wird von der Anmelderin unter der Bezeichnung NovaLung iLA (interventional Lung Assist) vertrieben.

Einen Überblick über neue Technologien für Lungenunterstützungssysteme gibt G. Matheis: ,,New technologies for respiratory assist" in Perfusion 2003; 18: 245-251. Matheis beschreibt u.a. das eingangs erwähnte NovaLung iLA, das auf einer Membranlunge beruht und für pulsatilen Blutfluss mit dichten Diffusionsmembranen ausgebildet ist, die avital mit einer Proteinmatrix beschichtet sind.

Blutseitig wird das NovaLung iLA unmittelbar an den Blutkreislauf eines Patienten über perkutane arterielle und venöse Kanulierung angeschlossen. Mit dem NovaLung iLA ist es ohne Verwendung einer blutseitigen Pumpe möglich, das von dem Herzen des Patienten pulsatil durch die Membranlunge gepumpte Blut von Kohlendioxid zu befreien und unter den Limitationen des arteriellen Bluteinstroms zu oxigenieren.

Über den ersten erfolgreichen Einsatz eines Vorgängers des Novalung iLA als Lungenunterstützungssystem berichten Reng et al.: ,,Pumpless extracorporeal lung assist and adult respiratory distress syndrome" in The Lancet 2000; 356: 219-220.

Die Bedeutung des NovaLung iLA sowie der erfindungsgemäßen künstlichen Lungensysteme ist vor dem Hintergrund zu sehen, dass Lungenerkrankungen nach den Statistiken der Weltgesundheitsorganisation die dritthäufigste Todesursache darstellen. Die mechanische Beatmung vermag zwar den Gasaustausch in fast allen Patienten aufrechtzuerhalten, erzeugt aber durch den unnatürlichen positiven Atemwegsdruck eine Schädigung der Lunge und anderer Organe, die als Ventilator Associated Lung Injury (VALI) bekannt ist. Mit dem NovaLung iLA ist es erstmals möglich, durch eine Ventilation außerhalb der Lunge eine sehr protektive Beatmung oder z.B. eine Überbrückung zur Lungentransplantation zu erzielen und den VALI zu vermeiden.

Es existiert jedoch kein chronisch einsetzbares Organersatzverfahren für die Lunge, wie es mit der Dialyse für das endgradige Nierenversagen der Fall ist, oder ein vollimplantierbares System, das die Lungenfunktion komplett ersetzen kann, wie es bei Herzunterstützungssystemen der Fall ist. Bei einem Lungenversagen besteht derzeit nur die Möglichkeit der maschinellen Beatmung, wobei es sich jedoch nicht um eine Lungenunterstützung handelt, denn die kranke Lunge wird nicht therapiert, vielmehr wird lediglich der lebensnotwendige Gasaustausch sichergestellt.

Sofern sie die streng gefassten Einschlußkriterien erfüllen, werden Patienten mit endgradig geschädigten Lungen daher für eine Lungentransplantation gelistet. Die Lungentransplantation stellt jedoch ein medizinisch extrem aufwändiges Verfahren dar, das zudem mit einem hohen Risiko für den Patienten verbunden ist. Abgesehen davon, dass dieses Therapiekonzept nur Patienten vorbehalten ist, die eine isolierte Lungenerkrankung haben und anderweitig gesund sind, sind die Langzeitergebnisse nicht zufriedenstellend. Hinzu kommt, dass auf Grund der geringen Menge der zur Verfügung stehenden Spenderorgane weltweit jährlich nur eine geringe Anzahl von Lungentransplantationen durchgeführt werden kann, die dem tatsächlichen Bedarf nicht genügt.

Vor diesem Hintergrund besteht ein signifikanter Bedarf an einem Lungenersatzverfahren als lebensverlängernde Behandlung (,,Destination Therapy"). Ein extrakorporales oder implantierbares Lungenunterstützungssystem, das sowohl das Blut oxygenieren als auch Kohlendioxid aus dem Blut entfernen kann, ist also für Patienten von allerhöchstem Wert. Derartige Lungenunterstützungssysteme können jedoch nicht nur bei Patienten, die nicht für eine Transplantation in Frage kommen, sondern auch bei Patienten angewendet werden, die auf eine Lungentransplantation warten, aber während der Wartezeit ein endgradiges Lungenversagen entwickeln, das zur Beatmung zwingt.

Dass künstliche Lungenunterstützungssysteme wie das NovaLung iLA hierfür prinzipiell einsetzbar sind, konnte in einer klinischen Studie gezeigt werden; siehe Fischer et al.: BRIDGE TO LUNG TRANSPLANTATION WITH THE NOVEL PUMPLESS INTERVENTIONAL LUNG ASSIST DEVICE NOVALUNG. in J Thorac Cardiovasc Surg. 2006;131(3):719-23.

Im Rahmen dieser Studie konnten Patienten, die trotz maschineller Beatmung ein beatmungsrefraktäres Lungenversagen entwickelten, mittels des Lungenunterstützungssystems NovaLung iLA erfolgreich zur Transplantation überbrückt werden. Derzeit können solche Systeme auf der Intensivstation für einen Zeitraum von wenigen Wochen eingesetzt werden, weil ein längerer Einsatz der NovaLung iLA durch eine Neointimabildung und andere Faktoren nicht möglich ist, außer man nimmt regelmäßig einen Austausch der Membranlunge vor.

Vor diesem Hintergrund ist es Aufgabe der vorliegenden Erfindung, das eingangs erwähnte künstliche Lungensystem derart weiterzubilden, dass es nicht nur zur kurzzeitigen Lungenunterstützung, sondern auch zur mittelfristigen und langfristigen extrakorporalen Lungenunterstützung oder zur Herstellung eines implantierbaren Lungenunterstützungssystems verwendet werden kann.

Erfindungsgemäß wird diese Aufgabe bei dem eingangs erwähnten künstlichen Lungensystem dadurch gelöst, dass die Fremdoberfläche auf der Blutseite und/oder der Luftseite mit biologischen Zellen besiedelt ist.

Die der Erfindung zugrundeliegende Aufgabe wird auf diese Weise vollkommen gelöst.

Die Erfinder der vorliegenden Anmeldung haben nämlich erkannt, dass die Neointimabildung und die Aktivierung inflammatorischer Reaktionen damit zusammenhängt, dass bei dem bekannten Lungenunterstützungssystem Fremdoberflächen vorliegen, die permanent mit dem Blut in Kontakt kommen. Nun sind zwar bei den bekannten Lungenunterstützungssystemen die Fremdoberflächen avital mit Protein und Heparin beschichtet, dennoch kommt es schon bei kurzzeitigem Einsatz zu systemischen Reaktionen (proinflammatorische Immunantwort), wie sie auch bei dem anderweitigen klinischen Einsatz von organunterstützenden Systemen mit Fremdoberflächen, die mit Blut in Berührung kommen, bekannt sind. Beispiele für derartige organunterstützende Systeme sind Herz-Lungen-Maschinen mit Oxygenator, mechanische Blutpumpen, Hämodialyse und Herzunterstützungssysteme, wie bspw. Kunstherzen. Neben dem Blutkontakt mit der Fremdoberfläche entstehen zudem durch mechanische Blutpumpen, wie sie bei allen bekannten Organunterstützungssystemen erforderlich sind, unphysiologische Scherkräfte mit entsprechender Blutschädigung.

Diese Probleme konnten mit der über den Blutkreislauf des Patienten perfundierten Kunstlunge NovaLung iLA überwunden werden, weil das System physiologisch vom Herzen des Patienten durchblutet wird, wobei das bekannte System dennoch nicht langfristig einsetzbar ist.

Erfindungsgemäß wird ein langfristig einsetzbares Lungenunterstützungssystem mit Zellen besiedelt, um erfolgreich funktionieren zu können, wobei diesen Zellen physiologische Perfusions- und Ventilationsbedingungen geboten werden können. Diese Anforderungen werden durch das erfindungsgemäße künstliche Lungensystem nun vollständig erfüllt.

Erfindungsgemäß wird somit eine Art Biohybridlunge bereitgestellt, die dem Ersatz bzw. der Unterstützung der Lungenfunktion dient. Dabei wird der Vorteil genutzt, dass individualisierte, zellularisierte Oberflächen die herkömmlich avital beschichteten Oberflächen ersetzen.

Auf diese Weise ist es möglich, eine kurzfristig oder langfristig einzusetzende künstliche Lunge herzustellen, die die Gasaustauschfunktion einer erkrankten Lunge vollends ersetzen kann, die also das Blut sowohl ausreichend oxygenieren kann und gleichfalls Kohlendioxid aus dem Blut entfernen kann.

Dabei ist es von Vorteil, wenn die Fremdoberflächen komplett mit biologischen Zellen, vorzugsweise autologen Zellen, besiedelt sind, um eine Fremdoberfläche auf der Blutseite und/oder auf der Luftseite zu vermeiden. Zu diesem Zweck wird die Blutseite mit Endothelzellen und die Luftseite mit alveolaren Epithelzellen (Pneumozyten) besiedelt.

Dabei ist es nicht unbedingt erforderlich, dass sowohl die Blutseite als auch die Luftseite mit biologischen Zellen besiedelt ist, die jeweils andere Seite kann auch avital beschichtet sein, bspw. mit Protein plus Heparin.

Wenn nur die Blutseite mit entsprechenden Endothelzellen besiedelt ist, die Fremdoberfläche dort also komplett von einem Zellrasen bedeckt ist, so treten die im Stand der Technik bekannten inflammatorischen Reaktionen nicht mehr auf, so dass schon aus diesem Grund das erfindungsgemäße künstliche Lungensystem eine deutlich längere Standzeit im Blutkreislauf eines Patienten aufweist als die bekannten Lungenunterstützungssysteme.

Wenn dagegen die Luftseite mit Pneumozyten besiedelt ist, findet eine Art biologische Verteidigung des Lungenunterstützungssystems statt, die Pneumozyten bilden also als Epithel die Barriere zum Individuum. Die Versorgung der Epithelzellen erfolgt über den Blutstrom, also durch die Gasaustauschmembran hindurch.

Die biologischen Zellen können dabei bspw. Stammzellen sein, Vorläuferzellen oder differenzierte Zellen. Als Quelle für Epithelzellen kommen dabei insbesondere embryonale Stammzellen, Stamm- oder Vorläuferzellen aus Nabelschnurblut, adulte mesenchymale Stammzellen, adulte Stammzellen, endotheliale Vorläuferzellen oder Endothelzellen in Betracht. Als Quelle für Pneumocyten sind insbesondere embryonale Stammzellen, Stamm- oder Vorläuferzellen aus Nabelschnurblut, adulte mesenchymale Stammzellen, adulte Stammzellen, pulmonale Vorläuferzellen, ausdifferenzierte Alveolarepithelzellen, insbesondere Typ I und II geeignet.

Insbesondere können die biologischen Zellen aus dem Respirationstrakt (Epithelzellen) oder einem Segment einer oberflächlichen Hautvene (Endothelzellen) entnommen und kultiviert werden. Erste Studien der Anmelderin haben aber ergeben, dass die biologischen Zellen z.B. auch aus Nabelschnurzellen gezüchtet werden können.

Bei dem erfindungsgemäßen künstlichen Lungensystem ist somit die Gasaustauschmembran eine Art Trennschicht, die entweder aus natürlichem oder künstlichem Material oder Mischungen davon hergestellt ist. Die eingesetzten Materialien können darüber hinaus biologisch abbaubar sein.

Erfindungsgemäß weist die Blutseite des künstlichen Lungensystems einen abgeschlossenen Blutraum mit Zu- und Abgängen zum Anschluss an einen natürlichen Blutkreislauf oder ein künstliches Perfusionssystem auf, wobei vorzugsweise die Luftseite einen abgeschlossenen Luftraum mit Zu- und Abgängen zum Anschluss an natürliche Atemwege oder ein künstliches Ventilationssystem aufweist.

Wenn das künstliche Lungensystem zur Herstellung eines extrakorporalen Lungenunterstützungssystems verwendet wird, wird der blutseitige Blutraum mit perkutaner Kanülierung oder mit subkutan gelegten Gefäßprothesen an eine Arterie sowie eine Vene angeschlossen, bspw. an die *A. subclavia* und die *V. subclavia.* Luftseitig ist dann ein künstliches Ventilationssystem vorgesehen, das den Luftraum physiologisch ventiliert, so dass die Pneumozyten wie beim natürlichen Einatmen mit Unterdruck belüftet werden.

Sofern das künstliche Lungensystem zur Herstellung eines implantierbaren Lungenunterstützungssystems verwendet wird, wird der Luftraum dagegen mit einem Zugang an die natürlichen Atemwege angeschlossen, bspw. an die Trachea bzw. die Bronchien. Ein Zugang des Luftraumes des künstlichen Lungensystems ist dann zum Anschluss an natürliche Atemwege ausgebildet ist, und der andere Zugang des Luftraumes kann mit einer Druckkammer verbunden werden, die über abwechselnde Expansion und Komprimierung einen Pendelluftstrom in dem Luftraum erzeugt.

Es ist jedoch auch möglich, das künstliche Lungensystem zur Herstellung eines Lungenmodellsystems für die Untersuchung der Toxizität von Pharmaka oder von Atemwegsbelastungen z.B. durch Schadstoffe zu verwenden, so dass es als Ersatz von Tiermodellsystemen bei toxikologischen Untersuchungsreihen dienen kann. In diesem Fall wird die Blutseite an ein künstliches Perfusionssystem, also bspw. einen künstlichen Blutkreislauf, angeschlossen, der so ausgelegt ist, dass die Endothelzellen physiologisch, also pulsatil, perfundiert werden. Die Luftseite wird dann an das künstliche Ventilationssystem angeschlossen, durch das die Pneumozyten physiologisch, also mit Unterdruck, ventiliert werden.

Zusätzlich weist das künstliche Ventilationssystem dann vorzugsweise einen Einlass für Fremdstoffe, wie flüchtige Substanzen oder Fremdgase, auf, deren Wirkung auf die Pneumozyten und/oder Endothelzellen getestet werden soll.

Dabei ist es nicht zwingend erforderlich, dass in dem künstlichen Perfusionssystem menschliches oder tierisches Blut zirkuliert, es kann auch Kunstblut oder ein sonstiges geeignetes Medium verwendet werden, über das die biologischen Zellen mit Nährstoffen versorgt werden.

Mit diesem Lungenmodellsystem kann dann die toxikologische Wirkung bspw. von flüchtigen Substanzen oder Fremdgasen auf die Funktion der Pneumozyten und Endothelzellen untersucht werden, ohne dass hierzu Tiermodellsysteme eingesetzt werden müssen.

Insgesamt ist es bei dem künstlichen Lungensystem von Vorteil, wenn die Gasaustauschmembran eine Diffusionsmembran ist, die vorzugsweise aus Polymethylpenten (PMP) besteht, wobei die Gasaustauschmembran auch als poröse oder mikroporöse Membran, bzw. Hohlfasermembran ausgebildet sein kann.

So können auch andere für Medizinprodukte einsetzbare und im Stand der Technik eingesetzte Membranen, z.B. Hämofiltrations- oder Dialysefasern, bei der Gasaustauschmembran eingesetzt werden.

Aufgabe der Gasaustauschmembran ist es zum einen, als Matrix für die Besiedelung mit Endothelzellen und Epithelzellen zu dienen, wobei sie ferner die Versorgung der Epithelzellen von der Blutseite her ermöglicht. Daher ist die Matrix dergestalt beschaffen, dass sie die pysiologischen Interaktionen von blutseitigen (Endothel) und luftseitigen (Pneumozyten) Zellen nicht behindert, während sie ein mechanisches Gerüst für diese Zellen bildet.

In ersten Studien konnte gezeigt werden, dass die Besiedelung der bereits klinisch zugelassenen Gasaustauschmembran aus Polymethylpenten mit Pneumozyten möglich ist, um die Fremdoberfläche in eine biologische Oberflächenstruktur umzuwandeln. Die dabei eingesetzten Pneumozyten wurden dadurch erzeugt, dass in humanen CD34⁺ blutbildenden Stammzellen (HSC), die aus Nabelschnurblut gewonnen wurden, die *in vitro*-Expression von endodermalem Phänotyp induziert wurde. Durch Kultivierung in Gegenwart des Wachstumsfaktors Activin A konnten HSC zum Phänotyp des distalen Lungenepithels differenziert werden.

Die Besiedelung der Blutseite mit Endothelzellen lässt sich mit gängigen Techniken erreichen, siehe bspw. XU C et al.: IN VITRO STUDY OF HUMAN VASCULAR ENDOTHELIAL CELL FUNCTION ON MATERIALS WITH VARIOUS SURFACE ROUGHNESS, IN J BIOMED MATER RES A. 2004 OCT 1;71(1):154-61.

Die Autoren beschreiben, dass sich humane vaskuläre Endothelzellen auf vorbehandelten, dreidimensionalen Stützstrukturen kultivieren lassen, die dazu dienen können, Blutgefäße mit kleinen Durchmessern zu ersetzen.

Bos et al. BLOOD COMPATIBILITY OF SURFACES WITH IMMOBILIZED ALBUMIN-HEPARIN CONJUGATE AND EFFECT OF ENDOTHELIAL CELL SEEDING ON PLATELET ADHESION, in: J BIOMED MATER RES. 1999 DEC 5;47(3):279-91, beschreiben die Besiedelung einer Albumin-Heparin Beschichtung mit Endothelzellen, die an der Beschichtung adhärieren und auf ihr proliferieren.

Bei der Verwendung von Polymethylpenten-Membranen ist ferner von Vorteil, dass diese bei einer reinen Endothelbeschichtung eine plasmadichte Schicht darstellt. Die im Stand der Technik beschriebenen, mit Endothelzellen beschichteten Membranen haben dagegen den Nachteil, dass sie einen Plasmadurchtritt auf die andere Seite der Membran ermöglichen, bzw. zur Vermeidung dieses Plasmadurchtritts aufwändig beschichtet werden müssen. Die PMP-Mambranen sind hingegen vorteilhafterweise plasmadicht.

In weiteren Ausführungsformen des künstlichen Lungensystems kann vorgesehen sein, dass die Gasaustauschmembran zusätzlich mit Substanzen beschichtet wird, die die Adhäsion und/oder Differenzierung der Zellen beeinflussen. Solche Faktoren sind bspw. Komponenten der Extrazellulären Matrix (ECM), wie bspw. Fibronektin, Laminin, Tenascin und Vitronektin, oder Wachstumsfaktoren, wie bspw. EGF (epidermal growth factor), FGF (fibroblast growth factor), GCSF, GGF (glia growth factor), GMCSF, GMA, GMF (glia maturation factor), IGF (insulin like growth factor), Interferone, Interleukine, Lymphokine, MCSF, Monokine, NGF (Nerve growth factor), NO (Stickstoffmonoxid), PD-ECGF (platelet derived endothelial cell growth factor), PDGF (platelet derived growth factor), TGF (transforming growth factor), TNF (Tumor-Nekrosis-Faktor), oder aber Antikörper, Nucleinsäuren, Aptamere, etc. , die entweder die Adhäsion und/oder Differenzierung von Zellen beeinflussen.

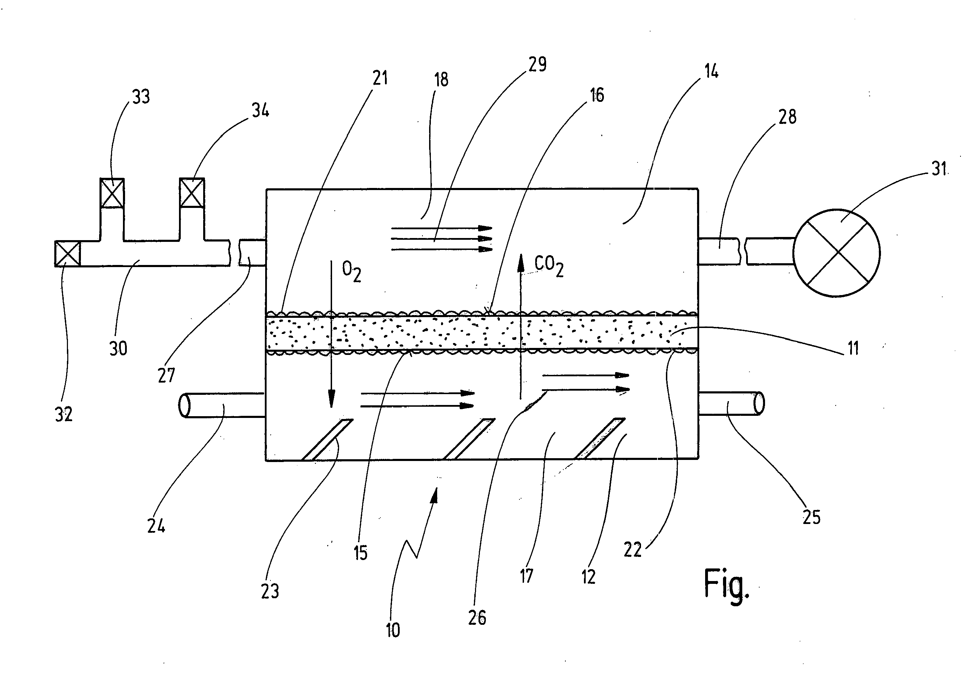

Weitere Vorteile ergeben sich aus der Beschreibung und der beigefügten Zeichnung.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert.

Die einzige Zeichnung zeigt das neue künstliche Lungensystem, das sowohl zur Herstellung eines extrakorporalen oder implantierbaren Lungenunterstützungssystem als auch zur Herstellung eines Lungenmodellsystems für die Untersuchung von Atemwegsbelastungen verwendet werden kann.

In der einzigen Figur ist mit 10 ein künstliches Lungensystem bezeichnet, das in der Figur äußerst schematisch dargestellt ist.

Das künstliche Lungensystem 10 umfasst eine Gasaustauschmembran 11, die eine Blutseite 12 von einer Luftseite 14 trennt. Die Gasaustauschmembran 11 weist mit ihren Fremdoberflächen 15 und 16 in einen abgeschlossenen Blutraum 17 auf der Blutseite 12 sowie einen abgeschlossenen Luftraum 18 auf der Luftseite 14 hinein.

Im Rahmen der vorliegenden Erfindung wird unter ,,Fremdoberfläche" eine künstliche Oberfläche verstanden, die *per se* nicht biokompatibel sein muss.

Die Gasaustauschmembran 11 ist im vorliegenden Fall eine Diffusionsmembran aus Polymethylpenten (PMP), wie sie in dem Lungenunterstützungssystem NovaLung iLA verwendet wird. Derartige PMP-Membranen können bspw. von der Firma Membrana Oehder Str. 28, D 42289 Wuppertal, unter der Bezeichung Oxyplus Kapillarmembran (Bestelnummer PMP 90/200) bezogen werden.

Die Gasaustauschmembran 11 ist eine Membran aus miteinander verwobenen Hohlfasern, wobei das Äußere der Hohlfasern der Blutseite 12 und das Innere der Hohlfasern der Luftseite 14 zugewandt ist. Diese geometrische Zuordnung ist in der Figur nicht dargestellt, vielmehr ist die Gasaustauschmembran 11 lediglich schematisch angedeutet.

Die Fremdoberfläche 16 im Luftraum 18 ist mit Epithelzellen 21 besiedelt, sie ist sozusagen vollständig von einem Alveolar- oder Pneumozytenrasen bedeckt.

Die Fremdoberfläche 15 in dem Blutraum 17 ist mit Endothelzellen 22 besiedelt, sie ist also sozusagen vollständig mit einem Endothelzellenrasen bedeckt.

In dem Blutraum 17 sind Blutanströmplatten 23 angeordnet, die dafür sorgen, dass zwischen einem venösen Anschluss 24 und einem arteriellen Anschluss 25 eine homogene Blutströmung 26 erzeugt wird, die für eine gleichmäßige Perfusion der Endothelzellen 22 sorgt.

Der Luftraum 18 ist mit einem Lufteinlass 27 sowie einem Luftauslass 28 verbunden, zwischen denen ein Luftstrom 29 zur Ventilation der Pneumozyten 21 erzeugt werden kann.

Der Luftauslass 28 kann bspw. mit einem Unterdrucksystem 31 verbunden werden, so dass die Ventilation des Luftraumes 18 physiologisch, also mit Unterdruck, erfolgt. Andererseits kann der Luftauslass 28 auch pulmonal angeschlossen werden, wobei der Lufteinlass 27 dann tracheal angeschlossen wird. Das Unterdrucksystem 31 kann auch eine Druckkammer 31 sein, die einen Pendelluftstrom erzeugt.

Schließlich ist es auch möglich, den Lufteinlass 27 mit einem Anschlussstück 30 zu verbinden, das einen Belüftungseingang 32, einen Fremdstoffeinlass 33 sowie einen Befeuchtungseinlass 34 aufweist.

Das künstliche Lungensystem 10 kann jetzt bspw. verwendet werden, um ein extrakorporales Lungenunterstützungssystem herzustellen.

Dazu wird der Luftauslass 28 mit dem Unterdrucksystem 31 und der Lufteinlass 27 mit einem in der Figur nicht gezeigten Fremdstofffilter verbunden, über den die angesogene Luft auch befeuchtet werden kann.

Der venöse Anschluss 24 wird z.B. über eine perkutane Kanüle mit einer Vene des Patienten verbunden, während der arterielle Anschluss 25 ebenfalls z.B. über eine perkutan gelegte Kanüle mit einer Arterie eines Patienten verbunden wird. Als Arterie und Vene können bspw. die *A*. und *V. subclavia* verwendet werden, zu denen ein subkutaner Anschluss des künstlichen Lungensystems über Gefäßprothesen erfolgt.

Auf diese Weise wird die Blutströmung 26 durch das Herz des Patienten bewegt, es ist also keine zusätzliche mechanische Pumpe erforderlich. Auf der Luftseite erfolgt eine Ventilation mit Unterdruck, so dass der Luftraum 18 physiologisch ventiliert wird.

Weil die Membran 11 jetzt auf der Luftseite 14 mit Pneumozyten 21 und auf der Blutseite 12 mit Endothelzellen 22 besiedelt ist, simuliert die künstliche Lunge 10 sozusagen die physiologische Situation, wobei die Pneumozyten 21 und Endothelzellen 22 physiologisch ventiliert bzw. mit z.B. dem Blut des Patienten perfundiert werden, so dass optimale Wachstums- und Funktionsbedingungen vorherrschen.

Das künstliche Lungensystem sorgt jetzt für eine Oxygenierung der Blutströmung 26, Sauerstoff gelangt also von dem Luftraum 18 in den Blutraum 17. Gleichzeitig wird Kohlendioxid aus dem Blutstrom 26 herausgezogen, CO₂ gelangt also aus dem Blutraum 17 in den Luftraum 18.

Nach Erkenntnissen der Erfinder der vorliegenden Anmeldung ist ein derartiges extrakorporales Lungenunterstützungssystem über lange Zeiträume einsetzbar, weil zum einen die Endothelzellen 22 sowie die physiologischen Strömungsbedingungen in dem Blutraum 17, die durch die Blutanströmplatte 23 noch unterstützt wird, jegliche Irritation des fließenden Blutes vermeidet, so dass die im Stand der Technik beobachteten Neointimabildung, Gerinnungsaktivierung und inflammatorische Reaktion etc. nicht mehr auftreten.

Da zusätzlich auf der Luftseite 14 Pneumozyten 21 die Fremdoberfläche 16 abdecken, erfolgt dort zum anderen eine biologische Verteidigung, die Pneumozyten stellen die physiologische Barriere zum Individuum Patient her, wobei die Pneumozyten durch die Blutströmung 26, also durch die Gasaustauschmembran 11 hindurch mit Nährstoffen versorgt werden. Zu diesem Zweck ist es erforderlich, die Gasaustauschmembran 11 mit einer hinreichenden Porengröße und Geometrie zu versetzen, um einerseits den Austausch von Nährstoffen und eine interzelluläre Kommunikation zu ermöglichen, andererseits aber den Durchtritt von Blut in den Luftraum 18 zu verhindern.

Alternativ kann das künstliche Lungensystem 10 auch zur Herstellung eines implantierbaren Lungenunterstützungssystems verwendet werden. In diesem Fall werden der venöse Anschluss 24 sowie der arterielle Anschluss 25 mit geeigneten Kanülen mit Venen und Arterien des Patienten innerhalb des Körpers verbunden. Der Lufteinlass 27 wird tracheal innerhalb des Körpers angeschlossen und an den Luftauslass wird bspw. eine mit-implantierte Druckkammer 31 angeschlossen, die entweder über eine externe mechanische Energiequelle oder über körpereigene Muskulatur abwechselnd expandiert und komprimiert wird. Hier wird also nur ein Anschluß an das Atemwegssystem vorgenommen und ein Pendelluftstrom erzeugt, der in natürlicher Weise die Biohybridlunge in bidirektionaler Durchströmung mit Ventilationsgas versorgt. Die Ventilation des Luftraumes 18 erfolgt also wie über die Atmungstätigkeit der Lunge des Patienten und die Perfusion des Blutraums 17 über die Herztätigkeit des Patienten, beides also physiologisch..

Bei einem implantierbaren Lungenunterstützungssystem dient der Lufteinlass 27 also dazu, den abgeschlossenen Luftraum 18 an die natürlichen Atemwege des Patienten anzuschließen.

Andererseits kann das künstliche Lungensystem 10 auch verwendet werden, um ein Lungenmodellsystem für die Untersuchung von Atemwegsbelastungen oder lungentoxischen Substanzen im Perfusat/Blutstrom herzustellen. In diesem Fall wird die Blutseite über den venösen Anschluss 24 und den arteriellen Anschluss 25 mit einem künstlichen Perfusionssystem verbunden, das einen künstlichen Blutkreislauf erzeugt, über den der Blutraum 17 physiologisch pulsatil perfundiert wird.

Der pulmonale Luftauslass 28 wird mit dem Unterdrucksystem 31 und der tracheale Lufteinlass 27 mit dem Anschlussstück 30 verbunden, so dass die Ventilation des Luftraumes 18 ebenfalls physiologisch, also mit Unterdruck, erfolgt. Die Pneumozyten 21 sowie die Endothelzellen 28 wachsen und leben also nach wie vor unter physiologischen Bedingungen.

Über den Fremdstoffeinlass 33 können jetzt bspw. flüchtige Substanzen oder Fremdgase in den Luftstrom 29 eingegeben werden, so dass im Rahmen von toxikologischen Untersuchungsreihen die Wirkung dieser Fremdstoffe auf die Pneumozyten 21 und Endothelzellen 22 untersucht werden kann. Dieses Lungenmodellsystem kann also die bisher verwendeten Tiermodellsysteme ersetzen, wenn bspw. die Toxizität oder maximale Arbeitsplatzkonzentration bestimmter Substanzen bestimmt werden soll.

Es sei noch erwähnt, dass für alle drei soeben beschriebenen Anwendungsfälle nicht unbedingt erforderlich ist, dass beide Fremdoberflächen 15 und 16 mit biologischen Zellen besiedelt sind, ein wesentlicher Vorteil bei allen drei Anwendungen wird bereits dann erreicht, wenn auf der Blutseite 12 die Fremdoberfläche 15 mit Endothelzellen 22 besiedelt ist.

Eine derartige Besiedelung lässt sich bspw. so erreichen, wie dies in den eingangs erwähnten Artikeln von Bos et al. sowie Xu et al. beschrieben ist, deren Inhalt hiermit durch Bezugnahme zum Gegenstand der vorliegenden Anmeldung gemacht wird.

In diesem Fall kann dann die Fremdoberfläche 16 auf der Luftseite 14 avital, bspw. mit Protein und Heparin, beschichtet sein.

Andererseits ist es auch möglich, nur die Fremdoberfläche 16 auf der Luftseite 14 mit Pneumozyten zu besiedeln und die Fremdoberfläche 15 auf der Blutseite 12 avital zu beschichten. Letzteres kann insbesondere dann sinnvoll sein, wenn im Rahmen eines Lungenmodellsystems der Blutraum 17 mit Kunstblut oder Medium durchströmt wird, das lediglich dazu verwendet wird, die Pneumozyten auf der Luftseite 14 mit Nährstoff zu versorgen.

Eine erste Pilotstudie hat ergeben, dass eine Besiedlung der aus PMP gefertigten Gasaustauschmembran 11 mit Pneumozyten möglich ist.

Zu diesem Zweck wurde die humane Typ II Pneumozyten-Tumorzelllinie A549 (American Type Culture Collection, Virginia, USA, # CCL 183; Lieber et al., Int. J. Cancer 1976; 17: 62-70), sowie die murine SV40-transformierte Typ II Pneumozytenzelllinie MLE 12 (American Type Culture Collection, Virginia, USA, # CRL 2110; Wikenheiser et al., PNAS USA 1993; 90: 11029-11033) mit 10 % (v/v) fötalem Kälberserum in Dulbecco's modifiziertem Eagle's Medium (DMEM, Invitrogen, Paisley, UK) kultiviert.

Kleine Stücke der PMP Fasermembran von ungefähr 1 cm x 0,5 cm wurden zugeschnitten, mit Bestrahlung durch UV-Licht sterilisiert und in das Kulturmedium gelegt. Die Pneumozyten wurden auf die Oberfläche der Fasern ausgesät und bei 37°C inkubiert.

Mit einem Inversionsmikroskop konnte das Zellwachstum beobachtet werden. Innerhalb weniger Tage hatten sich die Zellen auf der Oberfläche der Hohlfasern ausgebreitet und innerhalb von zwei Wochen wuchsen sie auch innerhalb der Fasern.

Diese ersten Experimente zeigen, dass es möglich ist, die Innenseiten von Hohlfasern mit Pneumozyten zu besiedeln.

Als Quelle für die Pneumozyten müssen dabei nicht unbedingt humane (A549) oder murine (MLE 12) Zelllinien verwendet werden, vielmehr können die Pneumozyten auch durch Differenzierung von humanen CD34⁺ blutbildenden Stammzellen (HSC) gezüchtet werden, die aus Nabelschnurblut gewonnen werden; siehe Albera et al.: "Human CD34+ Haematopoietic Stem Cells (HSC) From Umbilical Cord Blood Display An Endodermal Phenotype When Exposed To Activin A In Vitro", Blood 2005, 106:484A).

In diesen HSC konnte die *in vitro*-Expression von endodermalem Phänotyp durch Kultivierung in Gegenwart des Wachstumsfaktors Activin A induziert werden, die HSC konnten so zum Phänotyp des distalen Lungenepithels differenziert werden.

Obwohl die oben erwähnten Besiedelungstests mit robusten Pneumozyten-Zelllinien durchgeführt wurden, zeigen erste Ergebnisse der Anmelderin, dass auch aus Nabelschnurblut gezüchtete Pneumozyten, die in Gegenwart des Wachstumsfaktors Activin A zum Phänotyp des distalen Lungenepithels differenzierten, für die Besiedlung von PMP-Gasaustauschmembranen auf der Luftseite verwendet werden können.

## Patentansprüche

1. Künstliches Lungensystem mit einer Gasaustauschmembran (11), die eine Blutseite (12) von einer Luftseite (14) trennt, wobei die Gasaustauschmembran (11) auf der Blutseite (12) eine Fremdoberfläche (15) und auf der Luftseite (14) eine Fremdoberfläche (16) aufweist,
**dadurch gekennzeichnet, dass** die Fremdoberfläche (15) auf der Blutseite (12) und/oder die Fremdoberfläche (16) auf der Luftseite (14) mit biologischen Zellen (22, 21) besiedelt ist.

2. Künstliches Lungensystem nach Anspruch 1, **dadurch gekennzeichnet, dass** die Fremdoberfläche (15, 16) vollständig mit biologischen Zellen (22, 21) besiedelt ist.

3. Künstliches Lungensystem nach Anspruch 1 oder 2, **dadurch gekennzeichnet, dass** die Fremdoberfläche (15) auf der Blutseite (12) mit Endothelzellen (22) besiedelt ist.

4. Künstliches Lungensystem nach einem der Ansprüche 1 bis 3, **dadurch gekennzeichnet, dass** die Fremdoberfläche (16) auf der Luftseite (14) mit Alveolarepithelzellen (21) besiedelt ist.

5. Künstliches Lungensystem nach einem der Ansprüche 1 bis 4, **dadurch gekennzeichnet, dass** die nicht mit biologischen Zellen (21, 22) besiedelte Fremdoberfläche (15, 16) avital beschichtet ist.

6. Künstliches Lungensystem nach einem der Ansprüche 1 bis 5, **dadurch gekennzeichnet, dass** die Gasaustauschmembran (11) eine Diffusionsmembran ist.

7. Künstliches Lungensystem nach Anspruch 6, **dadurch gekennzeichnet, dass** die Gasaustauschmembran (11) zwischen Endothel und Pneumozyten aus Polymethylpenten (PMP) gefertigt ist.

8. Künstliches Lungensystem nach einem der Ansprüche 1 bis 7, **dadurch gekennzeichnet, dass** die Gasaustauschmembran (11) porös oder mikroporös ausgebildet ist.

9. Künstliches Lungensystem nach einem der Ansprüche 1 bis 8, **dadurch gekennzeichnet, dass** die Blutseite (12) einen abgeschlossenen Blutraum (17) mit Zu- und Abgängen (24, 25) zum Anschluss an einen natürlichen Blutkreislauf oder ein künstliches Perfusionssystem (31) aufweist.

10. Künstliches Lungensystem nach einem der Ansprüche 1 bis 9, **dadurch gekennzeichnet, dass** die Luftseite (14) einen abgeschlossenen Luftraum (18) mit Zugängen (27, 28) zum Anschluss an natürliche Atemwege oder ein künstliches Ventilationssystem (30, 31) aufweist.

11. Künstliches Lungensystem nach Anspruch 10, **dadurch gekennzeichnet, dass** das künstliche Ventilationssystem (30, 31) einen Einlass (33) für Fremdstoffe aufweist.

12. Künstliches Lungensystem nach einem der Ansprüche 1 bis 11, **dadurch gekennzeichnet, dass** die biologischen Zellen (21, 22) autologe Zellen sind, die vorzugsweise aus Respirationsextrakt, Segment einer oberflächigen Hautvene oder Nabelschnurzelle gewonnen werden.

13. Verwenden des künstlichen Lungensystems nach einem der Ansprüche 1 bis 12 zur Herstellung eines extrakorporalen Lungenunterstützungssystems.

14. Verwendung des künstlichen Lungensystems nach einem der Ansprüche 1 bis 12 zur Herstellung eines implantierbaren Lungenunterstützungssystems.

15. Verwendung nach Anspruch 14, **dadurch gekennzeichnet, dass** ein Zugang (27) des Luftraumes (18) des künstlichen Lungensystems zum Anschluss an natürliche Atemwege ausgebildet ist, und der andere Zugang (28) des Luftraumes (18) mit einer Druckkammer verbunden wird, die über abwechselnde Expansion und Komprimierung einen Pendelluftstrom in dem Luftraum (18) erzeugt.

16. Verwendung des künstlichen Lungensystems nach einem der Ansprüche 1 bis 12 zur Herstellung eines Lungenmodellsystems für die Untersuchung von Atemwegsbelastungen.
